# EUROPÄISCHE PATENTSCHRIFT

(11) **EP 1 575 927 B1**
(45) Veröffentlichungstag und Bekanntmachung des Hinweises auf die Patenterteilung: **04.08.2010**
(21) Anmeldenummer: 03813126.4
(22) Anmeldetag: 16.12.2003
(51) Int. Cl.: C07D 251/70

(54) **VERFAHREN ZUR HERSTELLUNG VON ALKOXYCARBONYLAMINO-TRIAZINEN**
METHOD FOR THE PRODUCTION OF ALKOXYCARBONYLAMINO TRIAZINES
PROCÉDÉ DE PRODUCTION D' ALCOXYCARBONYLAMINOTRIAZINES

(30) Priorität: 18.12.2002 DE 10259672
(43) Veröffentlichungstag der Anmeldung: 21.09.2005
(73) Patentinhaber: BASF SE, 67056 Ludwigshafen (DE)
(72) Erfinder: SCHNEIDER, Jörg, 69469 Weinheim (DE); SCHERR, Günter, 67065 Ludwigshafen (DE); SCHUPP, Hans, 67549 Worms (DE); EICHFELDER, Andreas, 67133 Maxdorf (DE); ROBERT, Alain, 67150 Niederkirchen (DE); REIF, Martin, 67354 Römerberg (DE)
(86) Internationale Anmeldenummer: PCT/EP2003/014274
(87) Internationale Veröffentlichungsnummer: WO 2004/054990

(56) Entgegenhaltungen:
- EP-A- 0 624 577
- DE-C- 92 535
- FINKE R G ET AL: "Model Studies of Coenzyme B12 Dependent Diol Dehydratase." JOURNAL OF THE AMERICAN CHEMICAL SOCIETY, AMERICAN CHEMICAL SOCIETY, WASHINGTON, DC, US, Bd. 105, Nr. 26, 1983, Seiten 7592-7604, XP002277223 ISSN: 0002-7863

## Beschreibung

Die vorliegende Erfindung betrifft ein neues Verfahren zur Herstellung von Alkoxycarbonylamino-triazinen durch Umsetzung von Di- oder Triaminotriazinen mit cyclischen Kohlensäureestern und gegebenenfalls mit untergeordneten Mengen von acyclischen Kohlensäureestern in Gegenwart eines Alkanols und eines Alkali- oder Erdalkalialkanolats als Base.

Aus der EP-A-624 577 ist die Herstellung von Alkoxycarbonylamino-triazinen durch Umsetzung von Triazinen, beispielsweise Melamin, mit acyclischen Kohlensäureestern in Gegenwart einer Base bekannt. In der Regel wird dort Melamin mit einem Kohlensäureester, z.B. Dimethylcarbonat, in Gegenwart des dem Kohlensäureester zugrundeliegenden Alkanols, hier z.B. Methanol, und in Gegenwart eines Alkalialkanolats, basierend auf dem Kohlensäureester zugrundeliegenden Alkanol, hier z.B. Methanol, als Base zur Reaktion gebracht. Es wird weiterhin beschrieben, Melamin z.B. mit Dimethylcarbonat in Gegenwart eines höheren Alkohols, beispielsweise Butanol oder 2-Ethylhexanol, und des entsprechenden Natriumalkanolats, hier z.B. Natriumbutanolat oder Natrium-(2-ethylhexanolat) als Base umzusetzen.

Dem Fachmann wird in der EP-A-624 577 die Lehre vermittelt, dass das dort beschriebene Verfahren ausschließlich mit acyclischen Kohlensäureestern durchgeführt werden kann.

Aufgabe der vorliegenden Erfindung war es nun, ein neues Verfahren zur Herstellung von Alkoxycarbonylamino-triazinen bereitzustellen, das einfach durchzuführen ist und das die Herstellung eines großen Spektrums von Mischungen von gemischt funktionalisierten und/oder isomeren Alkoxycarbonylamino-triazinen in hoher Ausbeute und Reinheit mittels der industriell leicht zugänglichen cyclischen Kohlensäureester erlaubt.

Es wurde nun gefunden, dass die Herstellung von Alkoxycarbonylamino-triazinen der Formel I. 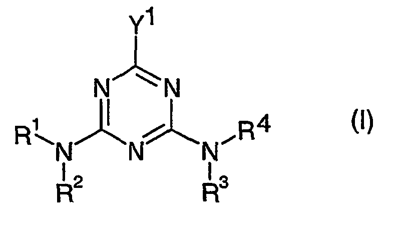 in der
- Y¹: Wasserstoff, C₁-C₄-Alkyl, gegebenenfalls durch C₁-C₄-Alkyl, C₁-C₄-Alkoxy oder Halogen substituiertes Phenyl oder einen Rest der Formel NR⁵R⁶ und
- R¹, R², R³, R⁴, R⁵ und R⁶: unabhängig voneinander jeweils Wasser- stoff oder einen Rest der Formel COOX oder X, worin X für C₁-C₁₃-Alkyl, dessen Kohlenstoffgerüst durch 1 oder 2 Sauer- stoffatome in Etherfunktion unterbrochen und/oder durch Hydroxy substituiert sein kann, oder C₃-C₆-Alkenyl steht, bedeuten,
mit der Maßgabe, dass in Formel I mindestens einer der Reste R¹ bis R⁴, oder wenn Y¹ für NR⁵R⁶ steht, mindestens einer der Reste R¹ bis R⁶ COOX bedeutet,
durch Umsetzung eines Triazins der Formel II 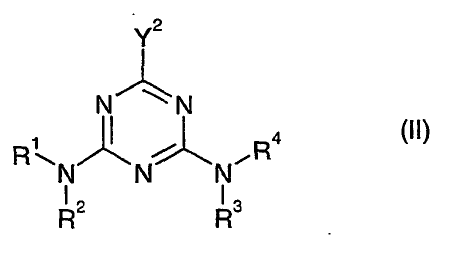 in der
- Y²: Wasserstoff, C₁-C₄-Alkyl, Amino oder gegebenenfalls durch C₁-C₄-Alkyl, C₁-C₄-Alkoxy oder Halogen substituiertes Phenyl bedeutet, und
- R¹ bis R⁴: jeweils die obengenannte Bedeutung besitzen,
mit der Maßgabe, dass in Formel II, wenn Y² nicht für Amino steht, mindestens einer der Reste R¹ bis R⁴ Wasserstoff bedeutet,
mit Kohlensäureestern in Gegenwart eines Alkohols und einer Base vorteilhaft gelingt, wenn man das Triazin der Formel II mit einem cyclischen Kohlensäureester der Formel III 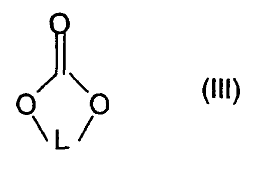 in der
- L: Ethylen, 1,2- oder 1,3-Propylen oder 1,2-, 1,4- 2,3-, oder 1,3-Butylen bedeutet,
sowie gegebenenfalls mit untergeordneten Mengen eines acyclischen Kohlensäureesters der Formel IV Z¹O-CO-OZ² (IV), in der
- Z¹ und Z²: unabhängig voneinander jeweils C₁-C₈-Alkyl bedeuten,
und einem C₁-C₁₃-Alkanol, dessen Kohlenstoffgerüst durch 1 oder 2 Sauerstoffatome in Etherfunktion unterbrochen und/oder durch Hydroxy substituiert sein kann, oder einem C₃-C₆-Alkenol in Gegenwart eines Alkali- oder Erdalkalialkanolats als Base umsetzt.

Alle in den hier aufgeführten Formeln enthaltenen Alkylreste können sowohl geradkettig als auch verzweigt sein.

Reste Y¹, Y², X, Z¹ und Z² sind z.B. Methyl, Ethyl, Propyl, Isopropyl, Butyl, Isobutyl, sec-Butyl oder tert-Butyl.

Reste X, Z¹ und Z² sind weiterhin z.B. Pentyl, Isopentyl, Neopentyl, tert-Pentyl, Hexyl, 2-Methylpentyl, Heptyl, Octyl, 2-Ethylhexyl und Isooctyl.

Reste X sind weiterhin z.B. Nonyl, Isononyl, Decyl, Isodecyl, Undecyl, Dodecyl, Tridecyl, Isotridecyl, 2-Methoxyethyl, 2-Ethoxyethyl, 2-Propoxyethyl, 2-Butoxyethyl, 2- oder 3-Methoxypropyl, 2- oder 3-Ethoxypropyl, 2- oder 3-Propoxypropyl, 2- oder 4-Methoxybutyl, 2- oder 4-Ethoxybutyl, 3,6-Dioxaheptyl, 3,6-Dioxaoctyl, 3,7-Dioxaoctyl, 4,7-Dioxaoctyl, 2- oder 3-Butoxypropyl oder 2-4-Butoxybutyl, 2-Hydroxyethyl, 2- oder 3-Hydroxypropyl, 2- oder 4-Hydroxybutyl, 3-Hydroxybut-2-yl, Allyl, Methallyl, Ethallyl, 2-, 3- oder 4-Penten-1-yl oder 2-, 3-, 4- oder 5-Hexen-1-yl. (Die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmann's Encyclopedia of Industrial Chemistry, 5th Edition, Vol. A 1, Seiten 290 bis 293, sowie Vol. A 10, Seiten 284 und 285.)

Reste Y¹ und Y² sind weiterhin z.B. Phenyl, 2-, 3- oder 4-Methylphenyl, 2-, 3- oder 4-Ethylphenyl, 2,4-Dimethylphenyl, 2-, 3- oder 4-Methoxyphenyl, 2-, 3- oder 4-Ethoxyphenyl, 2,4-Dimethoxyphenyl, 2-, 3- oder 4-Fluorphenyl oder 2-, 3- oder 4-Chlorphenyl.

Geeignete Alkanole, die im erfindungsgemäßen Verfahren zur Anwendung gelangen können, sind beispielsweise Methanol, Ethanol, Propanol, Isopropanol, Butanol, Isobutanol, sec-Butanol, tert-Butanol, Pentanol, Isopentanol, Neopentanol, tert-Pentanol, Hexanol, 2-Methylpentanol, Heptanol, Octanol, 2-Ethylhexanol, Isooctanol, Nonanol, Isononanol, Decanol, Isodecanol, Undecanol, Dodecanol, Tridecanol, Isotridecanol, 2-Methoxyethanol, 2-Ethoxyethanol, 2-Propoxyethanol, 2-Butoxyethanol, 2- oder 3-Methoxypropanol, 2- oder 3-Ethoxypropanol, 2- oder 3-Propoxypropanol, 2- oder 4-Methoxybutanol, 2- oder 4-Ethoxybutanol, 3,6-Dioxaheptanol, 3,6-Dioxaoctanol, 3,7-Dioxaoctanol, 4,7-Dioxaoctanol, 2- oder 3-Butoxypropanol, 2- oder 4-Butoxybutanol, Ethan-1,2-diol, Propan-1,2-diol, Propan-1,3-diol, 3-Oxa-5-hydroxypentanol, 3,6-Dioxa-8-hydroxyoctanol, 3-Oxa-5-hydroxy-2,5-dimethylpentanol oder 3,6-Dioxa-8-hydroxy-2,5,8-trimethyloctanol zu nennen.

Geeignete C₃-C₆-Alkenole, die im erfindungsgemäßen Verfahren zur Anwendung gelangen können, sind beispielsweise Allylalkohol, Methallylalkohol, Ethallylalkohol, 2-, 3- oder 4-Penten-1-ol oder 2-, 3-, 4- oder 5-Hexen-1-ol.

Bevorzugt ist die Verwendung von C₁-C₁₃-Alkanolen, wobei die Verwendung von C₁-C₇-Alkanolen besonders zu nennen ist.

Die im erfindungsgemäßen Verfahren verwendeten Alkohole können entweder einzeln oder auch als Mischungen untereinander zur Anwendung gelangen. Im letzteren Fall können die Anzahl der Mischungspartner sowie die Mischungsverhältnisse beliebig sein. Wenn in der Folge Alkanol oder Alkanolat erwähnt werden, so umfassen diese Begriffe auch die obengenannten Alkenole oder Alkenolate.

Geeignete Alkali- oder Erdalkalialkanolate, die erfindungsgemäß zur Anwendung gelangen können, sind z.B. die Lithium-, Natrium-, Kalium-, Magnesium- oder Calciumsalze der oben näher bezeichneten Alkanole. Die Verwendung von Alkalimethanolaten, insbesondere von Natriummethanolat ist bevorzugt.

Alkali- oder Erdalkalialkanolat kann entweder in festem Aggregatzustand oder in gelöster oder suspendierter Form zur Anwendung gelangen.

Bevorzugte Lösungsmittel/Verdünnungsmittel sind in diesem Fall insbesondere die oben näher bezeichneten Alkohole, allein oder als Mischung untereinander. Es können jedoch auch andere an sich bekannte und übliche inerte Verdünnungsmittel zur Anwendung gelangen.

Eine Verfahrensweise unter Verwendung eines Katalysators ist ebenfalls möglich.

Beispielsweise können Phasentransferkatalysatoren der Art, wie sie z.B. in Ullmann's Encyclopedia of Industrial Chemistry, 5th Edition, Vol. A 19, Seiten 239 bis 248, beschrieben sind, verwendet werden.

Weitere Katalysatoren können Metallsalze oder -komplexe sein, vorzugsweise Oxide, Chalkogenate, Carbonate oder Halogenide der Alkali-, Erdalkali- oder Übergangsmetalle. Zu nennen sind hier beispielsweise insbesondere Lithiumchlorid, Magnesiumchlorid oder Natriumcarbonat.

Im erfindungsgemäßen Verfahren kommen je Moläquivalent Aminogruppen im Triazin der Formel II in der Regel 1 bis 50 mol, vorzugsweise 3 bis 30 mol, Alkanol zur Anwendung.

Weiterhin kommen im erfindungsgemäßen Verfahren je Moläquivalent Aminogruppen im Triazin der Formel II in der Regel 0,1 bis 10 mol, vorzugsweise 1 bis 3 mol, cyclischer Kohlensäureester der Formel III zur Anwendung.

Weiterhin kommen im erfindungsgemäßen Verfahren je Moläquivalent Aminogruppen im Triazin der Formel II in der Regel 0,1 bis 10 Moläquivalent, vorzugsweise 1 bis 7 Moläquivalent, Alkali- oder Erdalkalialkanolat zur Anwendung.

Falls das erfindungsgemäße Verfahren in Gegenwart eines Katalysators durchgeführt wird, kommen im allgemeinen 10⁻¹⁰ bis 10 Gew.-%, vorzugsweise 10⁻³ bis 1 Gew.-%, Katalysator, jeweils bezogen auf das Gewicht des Triazins der Formel II, zur Anwendung.

Das erfindungsgemäße Verfahren wird im allgemeinen bei einer Temperatur von 20 bis 180°C, vorzugsweise 50 bis 120°C, durchgeführt.

Man arbeitet üblicherweise unter atmosphärischem Druck, wobei jedoch die Anwendung von erhöhtem Druck, in der Regel bis zu 8 bar, möglich ist.

Das erfindungsgemäße Verfahren wird mit cyclischen Kohlensäureestern der Formel III sowie gegebenenfalls mit untergeordneten Mengen an acyclischen Kohlensäureeestern durchgeführt. Untergeordnete Mengen im erfindungsgemäßen Sinn bedeutet, dass bis zu 30 mol-% der cyclischen Kohlensäureester der Formel III durch acyclische Kohlensäureeester der Formel IV ersetzt sein können.

Bevorzugt ist eine Verfahrensweise in der 0 bis 25 mol-%, vorzugsweise 0 bis 10 mol-% der cyclischen Kohlensäureester der Formel III durch acyclische Kohlensäureeester der Formel IV ersetzt sein können.

Falls im erfindungsgemäßen Verfahren auch acyclische Kohlensäureester der Formel IV zur Anwendung gelangen, sind solche bevorzugt, in der Z¹ und Z² unabhängig voneinander jeweils C₁-C₄-Alkyl bedeuten.

Von besonderem Interesse ist die Verwendung von Triazinen der Formel II, in der Y² Amino bedeutet als Edukt im erfindungsgemäßen Verfahren, wobei die Verwendung von Melamin (2,4,6-Triamino-1,3,5-triazin) ganz besonders hervorzuheben ist.

Von besonderem Interesse ist weiterhin die Verwendung eines cyclischen Kohlensäureester der Formel III, in der L Ethylen oder 1,2-Propylen, insbesondere Ethylen, bedeutet.

Von ganz besonderem Interesse ist die Herstellung von Alkoxycarbonylamino-triazinen der Formel V 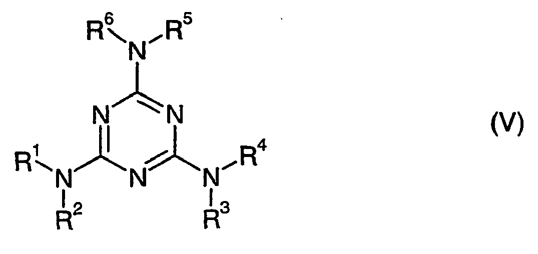 in der
R¹ bis R⁶ jeweils die obengenannte Bedeutung besitzen, mit der Maßgabe, dass drei dieser Reste jeweils Wasserstoff und die restlichen drei dieser Reste jeweils einen Rest der Formel COOX bedeuten, worin X die obengenannte Bedeutung besitzt, mittels des erfindungsgemäßen Verfahrens.

Die Herstellung der erfindungsgemäßen Alkoxycarbonylamino-triazinen kann in verschiedenen Varianten (A-F) erfolgen.

Vorteilhaft wird das erfindungsgemäße Verfahren so durchgeführt, dass man Triazin II und Alkanol vorlegt und dann in beliebiger Reihenfolge Alkali- oder Erdalkalialkanolat, in festem Zustand und/oder gelöst in Alkanol, und Kohlensäureester zudosiert, wobei die Dosierung von Alkali- oder Erdalkalialkanolat und Kohlensäureester vollständig vor Reaktionsbeginn oder teilweise vor Reaktionsbeginn und teilweise nach Reaktionsbeginn erfolgen kann. Durch Abdestillieren gewisser Mengen von Alkanol aus dem Reaktionsgemisch vor und/oder während der Reaktion kann die Einstellung gewünschter Alkanolverhältnisse bewirken.

In der Variante A) werden Triazin II, Alkanol und das gelöste Alkali- oder Erdalkalialkanolat zusammengegeben und anschließend bei erhöhter Temperatur (in der Regel 30 bis 85°C) Kohlensäureester hinzugegeben.

In Variante B) werden alle Komponenten vor Reaktionsbeginn vorgelegt.

In Variante C) werden Triazin II, Alkanol und Kohlensäureester vorgelegt und ein Teil des Alkali- oder Erdalkalialkanolats, das teilweise in gelöster und teilweise in festem Zustand vorliegt, vor Reaktionsbeginn und der Rest nach Reaktionsbeginn zudosiert. In Variante D) werden Triazin II, Alkanol und Kohlensäureester vorgelegt und ein Teil des Alkali- oder Erdalkalialkanolats, das entweder in gelöster Form oder in festem Zustand vorliegt, vor Reaktionsbeginn und der Rest nach Reaktionsbeginn zudosiert.

In Variante E) werden vor oder während der Reaktion Katalysatoren zugegeben.

In Variante F) werden vor oder während der Reaktion unterschiedliche Alkalialkanolate zugegeben (z.B. Lithiumalkanolat und Natriumalkanolat).

Die Reaktionsführung kann außerdem auch derart erfolgen, daß Melamin während der Reaktion stufenweise oder kontinuierlich zudosiert wird.

Das erfindungsgemäße Verfahren kann in üblichen Reaktionsapparaturen, z.B. einem Kessel- oder Rohrreaktor, durchgeführt werden. Wenn man das neue Verfahren so durchführt, dass das Molverhältnis Triazin der Formel II:Alkanol möglichst hoch ist, ist die Verwendung Apparaten mit Mischwirkung bei hochviskosen oder inhomogenen Reaktionsmischungen, z.B. Knetreaktoren, bevorzugt. Auch die Verwendung von selbstreinigenden Apparaten mit Mischwirkung ist möglich. Solche Apparate sind an sich bekannt und handelsüblich. Geeignete Reaktoren dieser Art sind z.B. der Kammerreaktor, der Kreislaufreaktor oder der Schneckenreaktor.

Vorteilhaft findet die Aufarbeitung des resultierenden Reaktionsgemisches in Abwesenheit zusätzlicher Lösungsmittel statt.

Dazu wird die alkanolische Reaktionsmischung direkt mit Säure, entweder durch Zudosieren von Säure oder durch Überführung der Reaktionsmischung in eine geeignete Säure, in Kontakt gebracht.

Die Säure kann dabei konzentriert zugegeben werden, und die Zugabe von Wasser während oder nach der Zudosierung der Säure erfolgen. Insbesondere bei Verwendung von wässrigen oder hochkonzentrierten Säuren muss während der Dosierung eine geeignete Durchmischung gewährleistet werden. Zum Ansäuern des Reaktionsgemisches können alle üblichen und industriell verfügbaren organischen und anorganischen Säuren in beliebiger Konzentration, vorzugsweise als 30 bis 85 gew.-%ige wässrige Lösungen, verwendet werden. Vorzugsweise verwendet man Mineralsäuren, deren Salze eine hohe Wasserlöslichkeit aufweisen, wie Salpetersäure, Schwefelsäure oder Phosphorsäure, aber auch die Carbonsäure Ameisensäure ist hier zu nennen.

Nach der Zugabe von Säure zum Reaktionsgemisch bilden sich eine wässrige, gegebenenfalls auch die Diole der Formel HO-L-OH, in der L die obengenannte Bedeutung besitzt, enthaltende Phase und eine alkanolische Phase, die von einander getrennt werden. Die Trennung der Phasen ist temperatur- und pH-Wert-abhängig, so dass die Zugabe von zusätzlichem Wasser bei einer Temperatur von 10 bis 70°C, bevorzugt von 15 bis 50°C, und bei einem pH-Wert von 0 bis 8, bevorzugt von 2 bis 5, erfolgt.

Die Zielprodukte resultieren direkt als 10 bis 80 gew.-%ige alkanolische Lösung. Durch anschließendes Einengen der alkanolischen Phase wird gleichzeitig mitgeschlepptes Wasser z. T. azeotrop (z.B. bei Butanol) entfernt, so dass weitere Trocknungsschritte, z.B. der Zusatz von Trocknungsmittel, nicht erforderlich sind.

Die Aufarbeitung des Reaktionsgemisches kann nach Neutralisation mit einer beliebigen Säure selbstverständlich auch durch Extraktion, Waschen und/oder durch Filtration erfolgen.

Das neue Verfahren, das sowohl in kontinuierlicher wie auch in diskontinuierlicher Arbeitsweise vorgenommen werden kann, liefert die Zielprodukte in hoher Ausbeute und Reinheit. Ein weiterer Vorteil des erfindungsgemäßen Verfahrens ist die Verwendung von cyclischen Kohlensäureestern, die gegenüber den acyclischen Kohlensäureestern sicherheitstechnisch als problemlos gelten.

Bei den mittels des erfindungsgemäßen Verfahrens erhältlichen Alkoxycarbonylamino-triazinen handelt es sich um wertvolle Lackrohstoffe.

Die folgenden Beispiele sollen die Erfindung näher erläutern.

Alle Reaktionen wurden unter Feuchtigkeitsausschluss durchgeführt. Bei Alkylcarbonylamino-triazin-Gemischen ließen sich die einzelnen Komponenten mittels HPLC (20 µl Schleife; UV-Detektor (250 nm); 1 ml/min, Acetonitril : wässr. Kalium-dihydrogenphosphat (0,05 mol/1) = 1:1; Säule Purospher-RP18e) trennen. Die Mengenangaben der Komponenten in den folgenden Beispielen erfolgt in Flächenprozent (A.-%). Die Stoffidentifikation erfolgte durch hochauflösende Massenspektrometrie, teilweise in Form einer direkten HPLC-MS-Kopplung oder mittels ¹H- und ¹³C-Kernresonanzspektroskopie.

### Beispiel 1

31,5 g (0,25 mol) Melamin, 1200 ml Butanol, 88,1 g (1 Mol) Ethylencarbonat und 94,5 g (1,75 mol) Natriummethanolat (fest) wurden bei einer Temperatur von 20°C vorgelegt. Anschließend wurde die Reaktionsmischung auf ca. 70°C erhitzt und weitere 120 Minuten bei ca. 70°C gerührt. Nach Abkühlen der Mischung auf ca. 30°C wurden unter Rühren 367,6 g (1,75 mol) wässrige Salpetersäure (30 gew.-%ig) zugegeben. Die wässrige Phase wurde abgetrennt und die organische homogene Phase weitere 3 mal mit je 300 ml Wasser gewaschen. Durch Einengen der organischen Phase resultierte eine 50 gew.-%ige butanolische Produktlösung, die überwiegend 2,4,6-Tris(butoxycarbonylamino)-1,3,5-triazin (30,2 A-%), 2-Methoxycarbonylamino-4,6-bis(butoxycarbonylamino)-1,3,5-triazin (35,5 A-%), 2,4-Bis(butoxycarbonylamino)-6-amino-1,3,5-triazin (7,3 A-%), 2,4-Bis(methoxycarbonylamino)-6-butoxycarbonylamino-1,3,5-triazin (12,1 A-%), 2-Butoxycarbonylamino-4-methoxycarbonylamino-6-amino-1,3,5-triazin (5,9 A-%) und Tris(methoxycarbonylamino)-1,3,5-triazin (4 A-%))enthielt (HPLC, ¹H-, ¹³C-NMR).

### Beispiel 2

Beispiel 2 wurde analog Beispiel 1 durchgeführt, jedoch betrug die Reaktionstemperatur 75°C. Nach 120 Minuten enthielt die Reaktionsmischung überwiegend 2,4,6-Tris(butoxycarbonylamino)-1,3,5-triazin (33,6 A-%), 2-Methoxycarbonylamino-4,6-bis(butoxycarbonylamino)-1,3,5-triazin (32,1 A-%), 2,4-Bis(butoxycarbonylamino)- 6-amino-1,3,5-triazin (9,1 A-%), 2,4-Bis(methoxycarbonylamino)-6-butoxycarbonylamino-1,3,5-triazin (8,8 A-%), 2-Butoxycarbonylamino-4-methoxycarbonylamino-6-amino-1,3,5-triazin (5,3 A-%) und Tris(methoxycarbonylamino)-1,3,5-triazin (4,5 A-%)

### Beispiel 3

31,5 g (0,25 mol) Melamin, 112 ml Butanol, 88,1 g (1 Mol) Ethylencarbonat und 151,3 g (1,75 mol) Natriummethanolat (fest) wurden bei einer Temperatur von 20°C in einen Knetreaktor (List-Reaktor) gefüllt. Anschließend wurde die Reaktionsmischung 1 Stunde bei 75°C geknetet. Durch direkte Analyse der Reaktionsmischung ließen sich die Hauptkomponenten der Mischung identifizieren als 2,4,6-Tris(methoxycarbonylamino)- 1,3,5-triazine, 2-Butoxycarbonylamino-4,6-bis(methoxycarbonylamino)-1,3,5-triazin und 2,4-Bis(butoxycarbonylamino)-6-methoxycarbonylamino-1,3,5-triazin, 2,4,6-Tris(butoxycarbonylamino)- 1,3,5-triazin, (HPLC, HPLC-MS).

### Beispiel 4

29 g (0,23 mol) Melamin, 976 ml Butanol, 81 g (0,92 mol) Ethylencarbonat und 87 g (1,61 mol) Natriummethanolat (fest) wurden bei einer Temperatur von 20°C vorgelegt. Anschließend wurde die Reaktionsmischung auf ca. 70 °C erhitzt und weitere 120 Minuten bei ca. 70°C gerührt. Nach Abkühlen der Mischung auf ca. 30°C wurden unter Rühren 338 g (1,61 mol) wässrige Salpetersäure (30 gew.-%ig) zugegeben. Die wässrige Phase wurde abgetrennt und die organische homogene Phase weitere 3 mal mit je 300 ml Wasser gewaschen. Durch Einengen der organischen Phase resultierte eine 50 gew.-%ige butanolische Produktlösung, die überwiegend 2,4,6-Tris(butoxycarbonylamino)-1,3,5-triazin (29,3 A-%), 2-Methoxycarbonylamino-4,6-bis(butoxycarbonylamino)-1,3,5-triazin (37,5 A-%), 2,4-Bis(butoxycarbonylamino)- 6-amino-1,3,5-triazin (6,3 A-%), 2,4-Bis(methoxycarbonylamino)-6-butoxycarbonylamino-1,3,5-triazin (13,2 A-%), 2-Butoxycarbonylamino-4-methoxycarbonylamino-6-amino-1,3,5-triazin (5,8 A-%) und Tris(methoxycarbonylamino)-1,3,5-triazin (4,7 A-%)enthielt (HPLC, ¹H-, ¹³C-NMR) .

### Beispiel 5

29,0 g (0,23 mol) Melamin, 1200 ml Butanol, 72,9 g (0,83 Mol) Ethylencarbonat 8,3 g (0,09 mol) Dimethylcarbonat und 87,0 g (1,61 mol) Natriummethanolat (fest) wurden bei einer Temperatur von 20°C vorgelegt. Anschließend wurde die Reaktionsmischung auf ca. 80°C erhitzt und weitere 120 Minuten bei ca. 80°C gerührt.
Nach Abkühlen der Mischung auf ca. 30°C wurden unter Rühren 338,2 g (1,61 mol) wässrige Salpetersäure (30 gew.-%ig) zugegeben. Die wässrige Phase wurde abgetrennt und die organische homogene Phase weitere 3 mal mit je 300 ml Wasser gewaschen. Durch Einengen der organischen Phase resultierte eine 50 gew.-%ige butanolische Produktlösung, die überwiegend 2,4,6-Tris(butoxycarbonylamino)-1,3,5-triazin (27,3 A-%), 2-Methoxycarbonylamino-4,6-bis(butoxycarbonylamino)-1,3,5-triazin (33,9 A-%), 2,4-Bis(butoxycarbonylamino)- 6-amino-1,3,5-triazin (6,9 A-%), 2,4-Bis(methoxycarbonylamino)-6-butoxycarbonylamino-1,3,5-triazin (12,6 A-%), 2-Butoxycarbonylamino-4-methoxycarbonylamino-6-amino-1,3,5-triazin (4,9 A-%) und Tris(methoxycarbonylamino)-1,3,5-triazin (8,0 A-%) enthielt (HPLC, ¹H-, ¹³C-NMR).

### Beispiel 6

Beispiel 6 wurde analog Beispiel 1 durchgeführt, jedoch wurden bei 50°C 1200 ml Butanol vorgelegt. Anschließend wurden 31,5 g (0,25 mol) Melamin, 110,0 g (1,25 Mol) Ethylencarbonat und 121,5 g (2,25 mol) Natriummethanolat (fest) zugegeben. Die Reaktionsmischung wurde anschließend 3 Stunden lang bei 70°C erhitzt. Die Reaktionsmischung enthielt insgesamt 88,4 % Triscarbonylaminotriazinverbindungen und ca. 9 % Dicarbonylaminotriazinverbindungen.

## Patentansprüche

1. Verfahren zur Herstellung von Alkoxycarbonylamino-triazinen
der Formel I 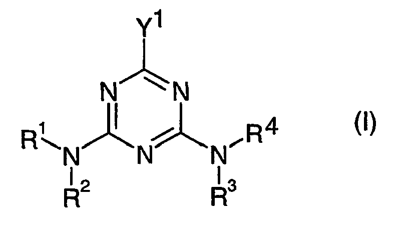 in der
Y¹ Wasserstoff, C₁-C₄-Alkyl, gegebenenfalls durch C₁-C₄-Alkyl, C₁-C₄-Alkoxy oder Halogen substituiertes Phenyl oder einen Rest der Formel NR⁵R⁶ und
R¹, R², R³, R⁴, R⁵ und R⁶ unabhängig voneinander jeweils Was- serstoff oder einen Rest der Formel COOX oder X, worin X für C₁-C₁₃-Alkyl, dessen Kohlenstoffgerüst durch 1 oder 2 Sauerstoffatome in Etherfunktion unterbrochen und/oder durch Hydroxy substituiert sein kann, oder C₃-C₆-Alkenyl steht, bedeuten,
mit der Maßgabe, dass in Formel I mindestens einer der Reste R¹ bis R⁴, oder wenn Y¹ für NR⁵R⁶ steht, mindestens einer der Reste R¹ bis R⁶ COOX bedeutet,
durch Umsetzung eines Triazins der Formel II 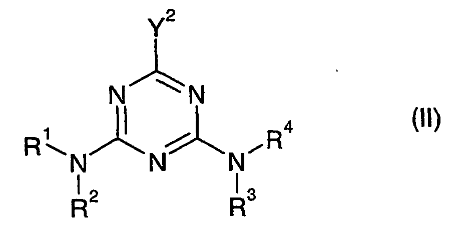 in der
Y² Wasserstoff, C₁-C₄-Alkyl, Amino oder gegebenenfalls durch C₁-C₄-Alkyl, C₁-C₄-Alkoxy oder Halogen substituiertes Phenyl bedeutet, und
R¹ bis R⁴ jeweils die obengenannte Bedeutung besitzen,
mit der Maßgabe, dass in Formel II, wenn Y² nicht für Amino steht, mindestens einer der Reste R¹ bis R⁴ Wasserstoff bedeutet,
mit Kohlensäureestern in Gegenwart eines Alkohols und einer Base, **dadurch gekennzeichnet, dass** man das Triazin der Formel
II mit einem cyclischen Kohlensäureester der Formel III 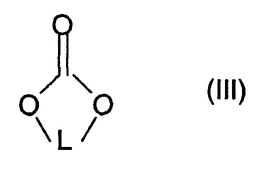
in der
L Ethylen, 1,2- oder 1,3-Propylen oder 1,2-, 1,4- 2,3-, oder 1,3-Butylen bedeutet,
sowie gegebenenfalls mit untergeordneten Mengen eines acyclischen Kohlensäureesters der Formel IV
Z¹O-CO-OZ² (IV),
in der
Z¹ und Z² unabhängig voneinander jeweils C₁-C₈-Alkyl bedeuten,
und einem C₁-C₁₃-Alkanol, dessen Kohlenstoffgerüst durch 1 oder 2 Sauerstoffatome in Etherfunktion unterbrochen und/oder durch Hydroxy substituiert sein kann, oder einem C₃-C₆-Alkenol in Gegenwart eines Alkali- oder Erdalkalialkanolats als Base umsetzt.

2. Verfahren nach Anspruch 1, **dadurch gekennzeichnet, dass** man ein C₁-C₁₃-Alkanol verwendet.

3. Verfahren nach Anspruch 1, **dadurch gekennzeichnet, dass** man ein Alkalialkanolat als Base verwendet.

4. Verfahren nach Anspruch 1, **dadurch gekennzeichnet, dass** man einen cyclischen Kohlensäureester der Formel III, in der L Ethylen oder 1,2-Propylen, bedeutet, verwendet.

5. Verfahren nach Anspruch 1, **dadurch gekennzeichnet, dass** man die Umsetzung bei einer Temperatur von 20 bis 180°C durchführt.

6. Verfahren nach Anspruch 1, **dadurch gekennzeichnet, dass** man die Umsetzung mit 1 bis 50 mol Alkanol, jeweils bezogen auf ein Moläquivalent Aminogruppen im Triazin der Formel II, vornimmt.

7. Verfahren nach Anspruch 1, **dadurch gekennzeichnet, dass** man die Umsetzung mit 0,1 bis 10 mol cyclischem Kohlensäureester, jeweils bezogen auf ein Moläquivalent Aminogruppen im Triazin der Formel II, vornimmt.

8. Verfahren nach Anspruch 1, **dadurch gekennzeichnet, dass** man die Umsetzung mit 0,1 bis 10 Moläquivalent Alkali- oder Erdalkalialkanolat, jeweils bezogen auf ein Moläquivalent Aminogruppen im Triazin der Formel II, vornimmt.

9. Verfahren nach Anspruch 1, **dadurch gekennzeichnet, dass** man dass man Triazin II und Alkanol vorlegt und dann in beliebiger Reihenfolge Alkali- oder Erdalkalialkanolat, in festem Zustand und/oder gelöst in Alkanol, und Kohlensäureester zudosiert, wobei die Dosierung von Alkali- oder Erdalkalialkanolat und Kohlensäureester vollständig vor Reaktionsbeginn oder teilweise vor Reaktionsbeginn und teilweise nach Reaktionsbeginn erfolgen kann.

10. Verfahren nach Anspruch 1, **dadurch gekennzeichnet, dass** 0 bis 25 mol-% der cyclischen Kohlensäureester der Formel III durch acyclische Kohlensäureeester der Formel IV ersetzt sein können.

## Claims

1. A process for preparing alkoxycarbonyl- aminotriazines of the formula I 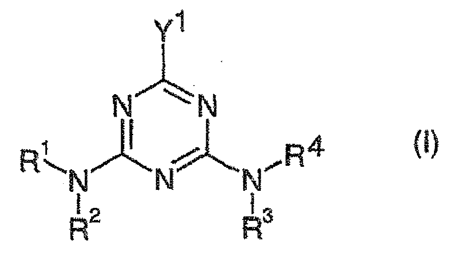 in which
Y¹ is hydrogen, C₁-C₄-alkyl, phenyl optionally substituted by C₁-C₄-alkyl, C₁-C₄-alkoxy or halogen, or a radical of the formula NR⁵R⁶ and
R¹, R², R³, R⁴, R⁵ and R⁶ are each independently hydrogen or a radical of the formula COOX or where X is C₁-C₁₃-alkyl whose carbon framework may be interrupted by 1 or 2 oxygen atoms in an ether function and/or be substituted by hydroxyl, or C₃-C₆-alkenyl,
with the proviso that at least one of the radicals R¹ to R⁴ in formula I or, when Y¹ is NR⁵R⁶, at least one of the radicals R¹ to R⁶ is COOX,
by reacting a triazine of the formula II 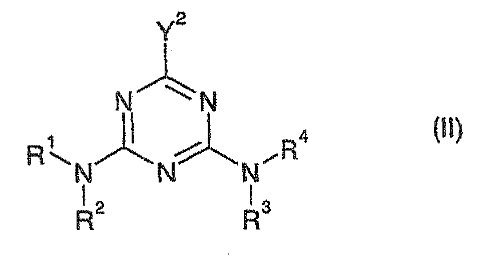 in which
Y² is hydrogen, C₁-C₄-alkyl, amino or phenyl optionally substituted by C₁-C₄-alkyl, C₁-C₄- alkoxy or halogen, and
R¹ to R⁴ are each as defined above,
with the proviso that, in formula II, when Y² is not amino, at least one of the radicals R¹ to R⁴ is hydrogen,
with carbonic esters in the presence of an alcohol and of a base, which comprises reacting the triazine of the formula II with a cyclic carbonic ester of the formula III 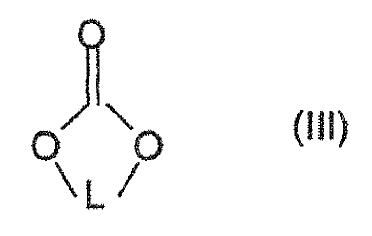 in which
L is ethylene, 1,2- or 1,3-propylene, or 1,2-, 1,4-, 2,3-, or 1,3-butylene,
and also optionally with minor amounts of an acyclic carbonic ester of the formula IV
Z¹O-CO-OZ² (IV),
in which
Z¹ and Z² are each independently C₁-C₈-alkyl,
and a C₁-C₁₃-alkanol whose carbon framework may be interrupted by 1 or 2 oxygen atoms in an ether function and/or be substituted by hydroxyl, or a C₃-C₆-alkenol, in the presence of an alkali metal alkoxide or alkaline earth metal alkoxide as a base.

2. The process according to claim 1, wherein a C₁-C₁₃-alkanol is used.

3. The process according to claim 1, wherein the base used is an alkali metal alkoxide.

4. The process according to claim 1, wherein a cyclic carbonic ester of the formula III in which L is ethylene or 1,2-propylene is used.

5. The process according to claim 1, wherein the reaction is carried out at a temperature of from 20 to 180_C.

6. The process according to claim 1, wherein the reaction is carried out with from 1 to 50 mol of alkanol, based in each case on one molar equivalent of amino groups in the triazine of the formula II.

7. The process according to claim 1, wherein the reaction is carried out with from 0.1 to 10 mol of cyclic carbonic ester, based in each case on one molar equivalent of amino groups in the triazine of the formula II.

8. The process according to claim 1, wherein the reaction is carried out with from 0.1 to 10 molar equivalents of alkali metal alkoxide or alkaline earth metal alkoxide, based in each case on one molar equivalent of amino groups in the triazine of the formula II.

9. The process according to claim 1, wherein triazine II and alkanol are initially charged and then, in any desired sequence, alkali metal alkoxide or alkaline earth metal alkoxide, in the solid state and/or dissolved in alkanol, and carbonic ester are metered in, and alkali metal alkoxide or alkaline earth metal alkoxide and carbonic ester can be metered in fully before the commencement of the reaction or partly before the commencement of the reaction and partly after the commencement of the reaction.

10. The process according to claim 1, wherein from 0 to 25 mol% of the cyclic carbonic esters of the formula III may be replaced by acyclic carbonic esters of the formula IV.

## Revendications

1. Procédé pour la préparation d'alcoxycarbonylaminotriazines de formule I 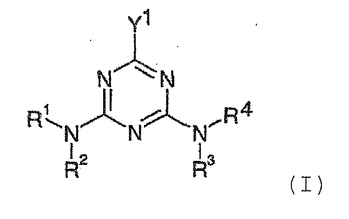 dans laquelle
Y¹ représente un atome d'hydrogène, un groupe alkyle en C₁-C₄, un groupe phényle éventuellement substitué par alkyle en C₁-C₄, alcoxy en C₁-C₄ ou halogène, ou un radical de formule NR⁵R⁶ et
R¹, R², R³, R⁴, R⁵ et R⁶ représentent chacun, indépendamment les uns des autres, un atome d'hydrogène ou un radical de formule COOX ou X, dans lequel X représente un groupe alkyle en C₁-C₁₃, dont le squelette carboné peut être interrompu par 1 ou 2 atomes d'oxygène en fonction éther et/ou substitué par hydroxy, ou représente un groupe alcényle en C₃-C₆,
étant entendu que dans la formule I au moins l'un des radicaux R¹ à R⁴, ou lorsque Y¹ représente NR⁵R⁶, au moins l'un des radicaux R¹ à R⁶ représente COOX,
par mise en réaction d'une triazine de formule II 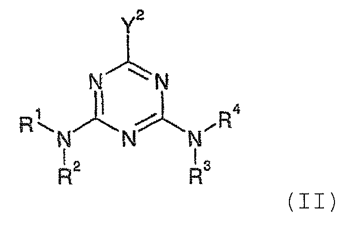 dans laquelle
Y² représente un atome d'hydrogène, un groupe alkyle en C₁-C₄, amino ou un groupe phényle éventuellement substitué par alkyle en C₁-C₄, alcoxy en C₁-C₄ ou halogène, et
R¹ à R⁴ ont chacun la signification indiquée plus haut,
étant entendu que dans la formule II, lorsque Y² ne représente pas un groupe amino, au moins l'un des radicaux R¹ à R⁴ représente un atome d'hydrogène,
avec des esters d'acide carbonique en présence d'un alcool et d'une base, **caractérisé en ce qu'**on fait réagir la triazine de formule II avec un ester cyclique d'acide carbonique de formule III 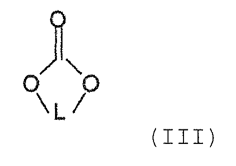 dans laquelle
L représente un groupe éthylène, 1,2- ou 1,3- propylène ou 1,2-, 1,4-, 2,3- ou 1,3-butylène,
ainsi qu'éventuellement avec des quantités minimes d'un ester acyclique d'acide carbonique de formule IV
Z¹O-CO-OZ² (IV),
dans laquelle
Z¹ et Z² représentent, indépendamment l'un de l'autre, chacun un groupe alkyle en C₁-C₈,
et un alcanol en C₁-C₁₃, dont le squelette carboné peut être interrompu par 1 ou 2 atomes d'oxygène en fonction éther et/ou substitué par hydroxy, ou un alcénol en C₃-C₆, en présence d'un alcanolate de métal alcalin ou alcalino-terreux en tant que base.

2. Procédé selon la revendication 1, **caractérisé en ce qu'**on utilise un alcanol en C₁-C₁₃.

3. Procédé selon la revendication 1, **caractérisé en ce qu'**on utilise comme base un alcanolate de métal alcalin.

4. Procédé selon la revendication 1, **caractérisé en ce qu'**on utilise un ester cyclique d'acide carbonique de formule III, dans laquelle L représente un groupe éthylène ou 1,2-propylène.

5. Procédé selon la revendication 1, **caractérisé en ce qu'**on effectue la réaction à une température de 20 à 180°C.

6. Procédé selon la revendication 1, **caractérisé en ce qu'**on effectue la réaction avec 1 à 50 moles d'alcanol, chaque fois par rapport à un équivalent molaire de groupes amino dans la triazine de formule II.

7. Procédé selon la revendication 1, **caractérisé en ce qu'**on effectue la réaction avec 0,1 à 10 moles d'ester cyclique d'acide carbonique, chaque fois par rapport à un équivalent molaire de groupes amino dans la triazine de formule II.

8. Procédé selon la revendication 1, **caractérisé en ce qu'**on effectue la réaction avec 0,1 à 10 équivalents molaires d'alcanolate de métal alcalin ou alcalino-terreux, chaque fois par rapport à un équivalent molaire de groupes amino dans la triazine de formule II.

9. Procédé selon la revendication 1, **caractérisé en ce qu'**on dispose au préalable la triazine II et l'alcanol et on ajoute ensuite par addition dosée, en un ordre quelconque, l'alcanolate de métal alcalin ou alcalino-terreux, à l'état solide et/ou dissous dans un alcanol, et l'ester d'acide carbonique, l'addition dosée d'alcanolate de métal alcalin ou alcalino-terreux et d'ester d'acide carbonique pouvant s'effectuer totalement avant le début de la réaction ou en partie avant le début de la réaction et en partie après le début de la réaction.

10. Procédé selon la revendication 1, **caractérisé en ce que** 0 à 25 % en moles de l'ester cyclique d'acide carbonique de formule III peuvent être remplacés par des esters acycliques d'acide carbonique de formule IV.
